# EUROPEAN PATENT APPLICATION

(11) **EP 4 556 039 A1**
(43) Date of publication of application: **21.05.2025**
(21) Application number: 23838688.2
(22) Date of filing: 27.06.2023
(51) Int. Cl.: A61M 11/00, A61M 15/00

(54) **ATOMIZATION-INHALATION-TYPE ATOMIZATION DRUG ADMINISTRATION DEVICE**

(30) Priority: 12.07.2022 CN 202210815658
(71) Applicant: Cansino Biologics Inc., Tianjin 300457 (CN)
(72) Inventor: SI, Weixue, Tianjin 300457 (CN); ZHU, Tao, Tianjin 300457 (CN); ZHAO, Xiaolong, Tianjin 300457 (CN); SU, Rongyao, Tianjin 300457 (CN); WANG, Yueran, Tianjin 300457 (CN); JIANG, Yueying, Tianjin 300457 (CN); LI, Ruoxi, Tianjin 300457 (CN); ZHU, Ali, Tianjin 300457 (CN); SHI, Wei, Tianjin 300457 (CN); PENG, Shaodan, Tianjin 300457 (CN)
(74) Representative: Finnegan Europe LLP
(86) International application number: PCT/CN2023/102859
(87) International publication number: WO 2024/012194

(57) **Abstract**

The present disclosure relates to a nebulizing-while-inhaling nebulization administration device. The nebulization administration device includes a quantitative administration control assembly, a drug storage assembly, a nebulization assembly, and a power supply assembly. The drug storage assembly is arranged in the upper housing (9) and is configured for providing a drug liquid for the nebulization assembly. The nebulization assembly is arranged between the upper housing (9) and the lower housing (2) and is configured for nebulizing the drug liquid provided by the drug storage assembly. The administration control assembly is configured for controlling operation of the nebulization assembly. The power supply assembly is configured for supplying power to the administration control assembly and the nebulization assembly. A nebulization outlet is arranged on the lower housing (2) and is configured for discharging the nebulized drug liquid. The nebulization administration device can store and nebulize a liquid pharmaceutical formulation and allows the nebulized liquid pharmaceutical formulation to be inhaled by a patient in real time, thereby achieving such an effect as treatment or immunization.

## Description

### TECHNICAL FIELD

The present disclosure relates to the field of medical devices, particularly to a nebulizing-while-inhaling nebulization administration device, and more particularly to a nebulizing-while-inhaling nebulization administration device for vaccine delivery.

### BACKGROUND

Nowadays, due to increasingly severe air pollution and sharp temperature fluctuations during seasonal transitions, the number of patients suffering from respiratory diseases has significantly increased.

One treatment method for respiratory diseases is the nebulization and inhalation administration, in which a nebulizer is utilized to disperse a drug liquid into fine droplets, and after the droplets are inhaled by a patient through the nose or mouth, the nebulized drug can directly act on the target organ, thereby increasing the local drug concentration and achieving a therapeutic effect. The nebulization and inhalation administration is very effective in treating bronchiolitis, asthmatic bronchitis, bronchial asthma, acute and chronic bronchitis, acute laryngitis, acute pneumonia, etc.

Inhalable nebulized vaccines are vaccines for immunization through nebulization and inhalation. The so-called nebulization and inhalation immunization refers to using a nebulizer to nebulize the vaccine into fine particles and allowing them to enter the respiratory tract and the lungs through inhalation to excite mucosal immunity. To prevent cross-infection during immunization inoculation, the device part through which a subject inhales must either be disinfected or discarded after use. Given the necessity of mass inoculation, there is an urgent need to provide a nebulization administration device that can not only prevent cross-infection but also reduce drug waste and has high inhalation inoculation efficiency.

Existing nebulization administration devices primarily operate in a nebulizing-while-inhaling mode, and subjects need instructions before administration. Both children and many adults often fail to properly control the frequency of inhalation administration and breathing, which significantly impacts the inhalation and absorption of the nebulized drug and can be very unfavorable for immunization inoculation or disease treatment; meanwhile, the nebulizing-while-inhaling mode may involve a risk of cross-infection. In addition, the curvature of the connection between the mouthpiece and the nebulizer body of existing nebulizers also directly or indirectly influences the nebulization effect. The mist resulting from nebulization collides with the corner of the connection between the housing of the nebulizer and the mouthpiece during inhalation, which can cause drug liquid aerosol deposition at the corner and thus a reduction in the inhalable drug amount. Therefore, the problem urgently needs to be resolved.

### SUMMARY

To overcome the defects of the prior art, the present disclosure provides a nebulizing-while-inhaling nebulization administration device. The nebulization administration device can be used to generate a drug mist and allow the drug mist to be inhaled by a subject to achieve such an effect as treatment or immunization, thereby resolving the problems of cross-infection, high costs, low efficiency, and drug liquid waste that are associated with existing nebulizing-while-inhaling nebulization devices.

To achieve the above objects, the present disclosure provides the following technical solutions:

Provided is a nebulization and inhalation device including a drug storage assembly, an administration control assembly, a nebulization assembly, and a power supply assembly;
preferably, the nebulization and inhalation device further includes an upper housing and a lower housing;
the drug storage assembly is arranged in the upper housing and is configured for providing a drug liquid for the nebulization assembly;
the nebulization assembly is arranged between the upper housing and the lower housing and is configured for nebulizing the drug liquid provided by the drug storage assembly;
the administration control assembly is configured for controlling operation of the nebulization assembly;
the power supply assembly is configured for supplying power to the administration control assembly and the nebulization assembly;
a nebulization outlet is arranged on the lower housing and is configured for discharging the nebulized drug liquid.

Further, the upper housing and the lower housing can be integrally formed, or the upper housing and the lower housing can be detachably connected. Furthermore, the upper housing and the lower housing are in a snap-fit connection.

Further, the drug storage assembly includes a liquid storage bottle arranged above the nebulization assembly.

Further, the drug storage assembly further includes a protective stopper configured for sealing the liquid storage bottle; for example, the liquid storage bottle may be sealed magnetically, through a snap-fit connection, through thread rotation, or the like. Furthermore, the protective stopper and the liquid storage bottle are separable and independent, or the protective stopper and the liquid storage bottle are connected through a connector to prevent the protective stopper from being lost.

Further, the nebulization assembly includes a vibrating mesh nebulizer arranged between the upper housing and the lower housing. Furthermore, the vibrating mesh nebulizer is arranged below the drug storage assembly.

Further, the administration control assembly includes an air inlet, an air switch for sensing airflow, and a control unit; the air inlet is arranged on the lower housing, and the air switch is arranged at the air inlet; the control unit receives a signal from the air switch to control operation of the nebulization assembly.

Further, the air switch is connected to the control unit and the nebulization assembly through wires. A change in the air pressure triggers the air switch for sensing airflow and is converted into a current, and the current is sent to the control unit and amplified. The control unit outputs a signal to control operation of the nebulization assembly.

Further, the air inlet is arranged at the bottom of the lower housing.

Further, the control unit is an MCU control unit. Furthermore, the control unit is arranged in the upper housing. Furthermore, the control unit is arranged in the upper housing and on a side of the liquid storage assembly.

Further, the administration control assembly further includes a driving vibrating mesh sheet operation indicator light configured for indicating whether a driving vibrating mesh sheet is operating or not. Furthermore, the operation indicator light is arranged on the MCU control unit.

Further, the power supply assembly includes a power interface or a power cable. The power supply assembly is electrically connected to the administration control assembly and the nebulization assembly and is configured for supplying power to the administration control assembly and the nebulization assembly. More preferably, the power supply assembly further includes a switch and a power indicator light; the switch is configured for controlling operation of the power supply assembly, and the power indicator light is configured for indicating whether the power supply assembly is operating or not.

Further, a side of the lower housing is provided with a mouthpiece interface; one side of the mouthpiece interface is connected to the lower housing, and the nebulization outlet is on the other side.

Further, the nebulization and inhalation device further includes an inhalation assembly. Furthermore, the inhalation assembly is a mouthpiece connected to the mouthpiece interface.

Further, the mouthpiece and the mouthpiece interface may be integrally formed or detachably connected. Furthermore, the mouthpiece is slidably inserted into the mouthpiece interface. Furthermore, the mouthpiece interface has a diameter of 10-30 mm, for example, 10, 15, 20, 25, or 30 mm.

Further, the connection between the lower housing and the mouthpiece interface has a right angle or is rounded. Further, when the connection between the lower housing and the mouthpiece interface is rounded, a chamfer radius R is 5-40 mm; preferably, R = 10-30 mm, for example, 5, 10, 15, 20, 25, 30, 35, or 40 mm.

Further, the vibrating mesh nebulizer is formed by attaching a piezoelectric ceramic element to a vibrating mesh sheet. The piezoelectric ceramic element can drive the vibrating mesh sheet to vibrate. The MCU control unit is electrically connected to the piezoelectric ceramic element.

Further, the nebulization assembly further includes an upper sealing ring and a lower sealing ring, and the vibrating mesh sheet is fixed and sealed through the upper sealing ring and the lower sealing ring.

Further, the administration control assembly further includes an air inlet blocking sheet and a blocking sheet support; the air inlet blocking sheet and the blocking sheet support are arranged at the air inlet, and the air inlet blocking sheet is mounted on the blocking sheet support; preferably, the air inlet blocking sheet is a one-way air intake device.

Further, the lower housing has a diameter of 10-40 mm, preferably 15-30 mm.

Further, the vibrating mesh sheet is made of a metal material or a thin film polymer material; preferably, the metal material is stainless steel or an alloy; more preferably, the metal material is a palladium alloy; preferably, the thin film polymer material is polypropylene (PP), polystyrene (PS), or polyimide (PI).

Further, the power interface is one of micro USB, USB Type-C, and Lightning interfaces.

The present disclosure can achieve the following beneficial technical effects:
1. The nebulizer of the present disclosure can be used to generate a drug mist and allow the drug mist to be inhaled by a subject to achieve such an effect as treatment or immunization.
2. The nebulizer of the present disclosure is an inhalation-driven nebulizer and can greatly improve inhalation efficiency, thereby reducing the drug amount needed to be added into the nebulizer.
3. When the connection between the lower housing and the mouthpiece interface in the nebulizer of the present disclosure has a chamfer radius of 5-40 mm, preferably 10-30 mm, as compared to when the connection has a right angle, the inhalable nebulized drug liquid amount is the highest, and a relatively good nebulization effect can be achieved to allow the drug liquid to be completely absorbed.

### BRIEF DESCRIPTION OF THE DRAWINGS

FIG. 1 is a schematic diagram of the structure of a nebulizing-while-inhaling nebulization administration device provided by the present disclosure.
FIG. 2 is an enlarged view of portion A in FIG. 1.

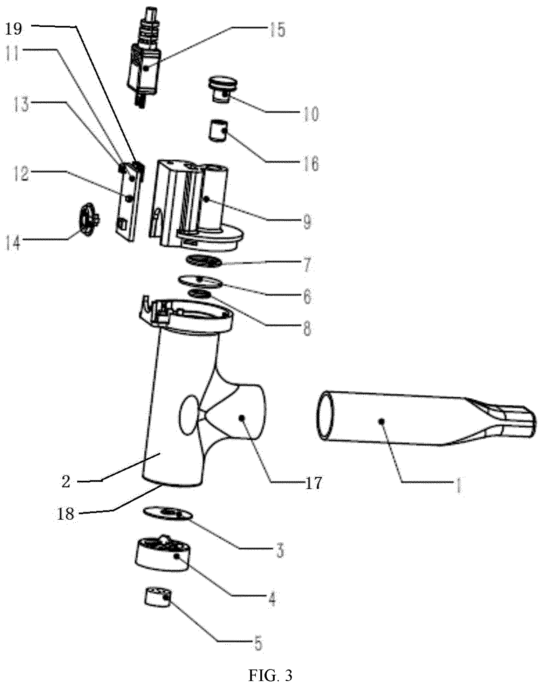

FIG. 3 is an exploded view of the structure of a nebulizing-while-inhaling nebulization administration device provided by the present disclosure.

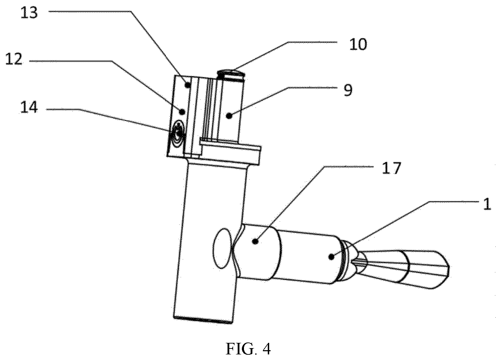

FIG. 4 is a cross-sectional view of the structure of a quantitative administration control device of a nebulizing-while-inhaling nebulization administration device provided by the present disclosure.

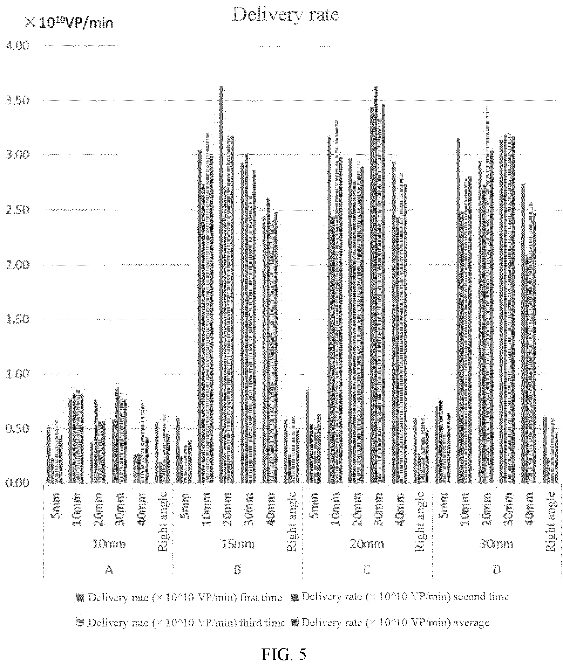

FIG. 5 is a bar graph of the relationship between different chamfer radii between the nebulizer and the mouthpiece and the delivery rate (each group is tested 3 times, with the results presented from left to right as follows: the first test result, the second test result, the third test result, and the average of the three test results).

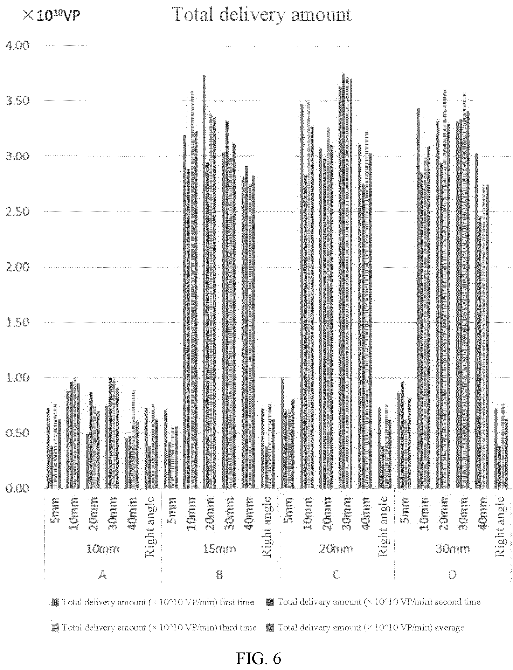

FIG. 6 is a bar graph of the relationship between different chamfer radii between the nebulizer and the mouthpiece and the total delivery amount (each group is tested 3 times, with the results presented from left to right as follows: the first test result, the second test result, the third test result, and the average of the three test results).

Reference numerals in the figures: 1. a mouthpiece; 2. a lower housing; 3. an air inlet blocking sheet; 4. a blocking sheet support; 5. an air switch for sensing airflow; 6. a vibrating mesh nebulizer; 7. an upper sealing ring; 8. a lower seal ring; 9. an upper housing; 10. a protective stopper; 11. an MCU control unit; 12. a power indicator light; 13. a driving vibrating mesh sheet operation indicator light; 14. a switch; 15. a power cable; 16. a drug storage bottle; 17. a mouthpiece interface; 18. an air inlet; and 19. a power interface.

### DETAILED DESCRIPTION

Unless otherwise defined, all scientific and technical terms used in the present disclosure have the same meaning as commonly understood by those skilled in the art to which the present disclosure relates.

The disclosures of the various publications, patents, and published patent specifications cited herein are hereby incorporated by reference in their entirety.

The technical solutions of the present disclosure are clearly and completely described below with reference to examples of the present disclosure; apparently, the described examples are only exemplary of the present disclosure instead of all examples of the present disclosure. Based on the examples of the present disclosure, all other examples obtained by those of ordinary skill in the art without creative effort shall fall within the protection scope of the present disclosure.

### Example 1

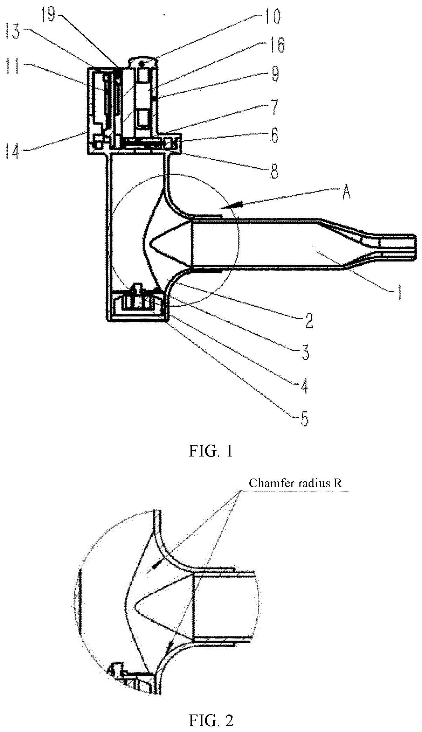

A nebulizing-while-inhaling nebulizer is shown in FIG. 1. The nebulizer includes an upper housing 9, a lower housing 2, a drug storage assembly, an administration control assembly, a nebulization assembly, a power supply device, and an inhalation assembly.

The upper housing 9 and the lower housing 2 are in a snap-fit connection.

The drug storage assembly includes a drug storage bottle 16 and a protective stopper 10, and the drug storage bottle 16 is located in the nebulizer upper housing 9. The detachable protective stopper 10 is mounted on top of the drug storage bottle 16 to facilitate the operation of adding a liquid to a drug storage bottle 15.

The nebulization assembly includes a vibrating mesh nebulizer 6, an upper sealing ring 7, and a lower sealing ring 8. The vibrating mesh nebulizer 6 is mounted between the nebulizer upper housing 9 and the nebulizer lower housing 2 and is fixed and sealed through the upper sealing ring 7 and the lower sealing ring 8.

The administration control assembly includes an air inlet 18, an air switch 5 for sensing airflow, an MCU control unit 11, and a driving vibrating mesh sheet operation indicator light 13. The air inlet 18 is arranged at the bottom of the nebulizer lower housing 2. The air inlet blocking sheet 3, the blocking sheet support 4, and the air switch 5 are all arranged at the air inlet 18 located at the bottom of the nebulizer lower housing 2. The air inlet blocking sheet 3 is mounted on the blocking sheet support 4 and is a one-way air intake device. The air switch 5 is mounted under the blocking sheet support 4 to sense changes in airflow. The MCU control unit 11 is mounted in the nebulizer upper housing 9 and is electrically connected to the air switch 5 and the vibrating mesh nebulizer 6. The driving vibrating mesh sheet operation indicator light 13 is mounted on the MCU control unit 11.

The power supply device includes a power interface 19, a switch 14, and a power indicator light 12. The power interface 19, the switch 14, and the power indicator light 12 are all mounted on the nebulizer upper housing 9 and are electrically connected to the MCU control unit 11. The power interface 19 is specifically a Micro USB interface and can be connected to a power cable 15 for power supply.

The inhalation assembly is a mouthpiece 1.

A side wall of the nebulizer lower housing 2 is provided with a mouthpiece interface 17, and the mouthpiece 1 is detachably connected to the nebulizer lower housing 2 through the mouthpiece interface 17. The connection between the nebulizer lower housing 2 and the mouthpiece interface 17 has a chamfer radius of 5-20 mm.

The nebulization and inhalation device according to the present disclosure operates as follows:

A specified dose of a drug liquid is added into the drug storage bottle 16 in the nebulizer upper housing 9. The device is connected to a power supply through the Micro USB interface of the power interface 19, and the switch 14 is pressed to turn on the power indicator light 12. When the user inhales through the mouthpiece 1, air passes through the air switch 5, waking up the MCU control unit 11, and the MCU control unit 11 immediately drives the vibrating mesh nebulizer 6 to spray the nebulized drug liquid and turns on the driving vibrating mesh sheet operation indicator light 13 at the top. When detecting that the airflow signal weakens or disappears, the MCU control unit 11 stops driving operation of the vibrating mesh sheet 6 and the operation indicator light 13.

The vibrating mesh nebulizer 6 is formed by attaching a piezoelectric ceramic element to a vibrating mesh sheet. The piezoelectric ceramic element can drive the vibrating mesh sheet to vibrate, and the MCU control unit 11 is electrically connected to the piezoelectric ceramic element.

### Example 2

The influence of nebulizers with different chamfer diameters between the nebulizer lower housing and the mouthpiece interface on the nebulization effect was investigated.

Experimental groups: Vibrating meshes made of a stainless steel material were selected and mounted in nebulizers that have nebulizer lower housings with a diameter of 10 mm, 15 mm, 20 mm, or 30 mm and forming a chamfer radius R of 5 mm, 10 mm, 20 mm, 30 mm, or 40 mm at the connection with the mouthpiece interface.

Model drug: recombinant SARS-CoV-2 vaccine (adenovirus 5 vector) liquid formulation (CanSino Biologics Inc.-Convidecia^{®}).

Specification: 0.5 mL per vial, containing 5 × 10¹⁰ VP of recombinant replication-deficient human adenovirus 5 expressing the S protein of SARS-CoV-2.

### Breathing simulator: BRS 200i

Experimental procedure: The respiratory mode was set to the adult mode. 0.5 mL of the vaccine drug liquid was added to the drug storage bottle of the nebulizer. The nebulizer was started, and the vaccine aerosol generated by the nebulizer was collected onto a matching glass fiber filter membrane through the breathing simulator. The virus particles on the filter membrane were eluted using an eluent, and the VP concentration in the eluate was determined using the ELISA method. The total number of virus particles adsorbed by the glass fiber filter membrane was calculated to evaluate the delivery rate and total delivery amount of Ad5-nCoV after nebulization. Table 1 shows values between the delivery rate and the total delivery amount for nebulizers with different chamfer radii between the nebulizer lower housing and the mouthpiece.

**Table 1**

| No. | Diameter | Radius | Delivery rate (× 10¹⁰ VP/min) | | | | Total delivery amount (× 10¹⁰ VP) | | | |
|---|---|---|---|---|---|---|---|---|---|---|
| | | | First time | Second time | Third time | Average | First time | Second time | Third time | Average |
| | 10mm | 5mm | 0.52 | 0.23 | 0.58 | 0.44 | 0.72 | 0.38 | 0.76 | 0.62 |
| | | 10mm | 0.77 | 0.82 | 0.87 | 0.82 | 0.88 | 0.96 | 1.00 | 0.95 |
| A | | 20mm | 0.38 | 0.77 | 0.57 | 0.57 | 0.49 | 0.87 | 0.74 | 0.70 |
| | | 30mm | 0.59 | 0.88 | 0.83 | 0.77 | 0.74 | 1.00 | 0.99 | 0.91 |
| | | 40mm | 0.26 | 0.27 | 0.75 | 0.43 | 0.45 | 0.47 | 0.89 | 0.60 |
| | | Right | 0.56 | 0.19 | 0.63 | 0.46 | 0.72 | 0.38 | 0.76 | 0.62 |
| | | angle | | | | | | | | |
| B | 15mm | 5mm | 0.60 | 0.24 | 0.35 | 0.40 | 0.71 | 0.41 | 0.55 | 0.56 |
| | | 10mm | 3.04 | 2.73 | 3.20 | 2.99 | 3.19 | 2.88 | 3.59 | 3.22 |
| | | 20mm | 3.63 | 2.71 | 3.18 | 3.17 | 3.73 | 2.94 | 3.38 | 3.35 |
| | | 30mm | 2.93 | 3.01 | 2.63 | 2.86 | 3.04 | 3.32 | 2.98 | 3.11 |
| | | 40mm | 2.44 | 2.60 | 2.41 | 2.48 | 2.81 | 2.91 | 2.75 | 2.82 |
| | | Right angle | 0.59 | 0.26 | 0.61 | 0.49 | 0.72 | 0.38 | 0.76 | 0.62 |
| C | 20mm | 5mm | 0.86 | 0.54 | 0.52 | 0.64 | 1.00 | 0.70 | 0.71 | 0.80 |
| | | 10mm | 3.17 | 2.45 | 3.32 | 2.98 | 3.47 | 2.83 | 3.48 | 3.26 |
| | | 20mm | 2.97 | 2.77 | 2.94 | 2.89 | 3.07 | 2.98 | 3.26 | 3.10 |
| | | 30mm | 3.44 | 3.63 | 3.34 | 3.47 | 3.63 | 3.74 | 3.72 | 3.70 |
| | | 40mm | 2.94 | 2.43 | 2.83 | 2.73 | 3.10 | 2.75 | 3.23 | 3.03 |
| | | Right angle | 0.6 | 0.27 | 0.61 | 0.49 | 0.72 | 0.38 | 0.76 | 0.62 |
| D | 30mm | 5mm | 0.71 | 0.76 | 0.46 | 0.64 | 0.86 | 0.96 | 0.62 | 0.81 |
| | | 10mm | 3.15 | 2.49 | 2.78 | 2.81 | 3.43 | 2.85 | 2.99 | 3.09 |
| | | 20mm | 2.95 | 2.73 | 3.45 | 3.04 | 3.32 | 2.94 | 3.60 | 3.29 |
| | | 30mm | 3.14 | 3.18 | 3.20 | 3.17 | 3.31 | 3.33 | 3.58 | 3.41 |
| | | 40mm | 2.74 | 2.09 | 2.57 | 2.47 | 3.03 | 2.45 | 2.74 | 2.74 |
| | | Right angle | 0.61 | 0.23 | 0.6 | 0.48 | 0.72 | 0.38 | 0.76 | 0.62 |

The results show that:
According to the delivery rate and total delivery amount results of the nebulization of 0.5 mL of the vaccine, when the chamfer radius R between the nebulizer lower housing and the nebulizer mouthpiece was 10 to 40 mm, a relatively high delivery rate and a relatively high total delivery amount were achieved, and a relatively good nebulization effect was achieved, which can result in a relatively high absorption efficiency of the nebulized drug liquid in the user.

The above descriptions are only preferred examples of the present disclosure and are not intended to limit the present disclosure. Any modifications, equivalent substitutions, etc. made without departing from the spirit and principle of the present disclosure shall fall within the protection scope of the present disclosure.

The foregoing examples and methods described in the present disclosure may vary depending on the abilities, experience, and preferences of those skilled in the art.

The specific order of the steps of the methods listed in the present disclosure does not constitute any limitation on the order of the steps of the methods of the present disclosure.

## Claims

1. A nebulizing-while-inhaling nebulization and inhalation device, comprising a drug storage assembly, an administration control assembly, a nebulization assembly, and a power supply assembly.

2. The nebulizing-while-inhaling nebulization and inhalation device according to claim 1, comprising an upper housing and a lower housing, wherein preferably, the drug storage assembly is arranged in the upper housing and is configured for providing a drug liquid for the nebulization assembly.

3. The nebulizing-while-inhaling nebulization and inhalation device according to claim 1, wherein the nebulization assembly is arranged between the upper housing and the lower housing and is configured for nebulizing the drug liquid provided by the drug storage assembly; and/or the administration control assembly is configured for controlling operation of the nebulization assembly; and/or the power supply assembly is configured for supplying power to the administration control assembly and the nebulization assembly; and/or a nebulization outlet is arranged on the lower housing and is configured for discharging the nebulized drug liquid.

4. The nebulizing-while-inhaling nebulization and inhalation device according to claim 1, wherein the drug storage assembly comprises a liquid storage bottle arranged above the nebulization assembly.

5. The nebulizing-while-inhaling nebulization and inhalation device according to claim 1, wherein the nebulization assembly comprises a vibrating mesh nebulizer arranged between the upper housing and the lower housing.

6. The nebulizing-while-inhaling nebulization and inhalation device according to claim 1, wherein the administration control assembly comprises an air inlet, an air switch for sensing airflow, and a control unit; wherein the air inlet is arranged on the lower housing, and the air switch is arranged at the air inlet; the control unit receives a signal from the air switch to control operation of the nebulization assembly.

7. The nebulizing-while-inhaling nebulization and inhalation device according to claim 6, wherein the administration control assembly further comprises an air inlet blocking sheet and a blocking sheet support; wherein the air inlet blocking sheet and the blocking sheet support are arranged at the air inlet, and the air inlet blocking sheet is mounted on the blocking sheet support; the air switch is mounted under the blocking sheet support.

8. The nebulizing-while-inhaling nebulization and inhalation device according to claim 1, wherein the power supply assembly comprises a power interface or a power cable, and wherein the power supply assembly is electrically connected to the administration control assembly and the nebulization assembly and is configured for supplying power to the administration control assembly and the nebulization assembly.

9. The nebulizing-while-inhaling nebulization and inhalation device according to claim 1, wherein a side of the lower housing is provided with a mouthpiece interface; wherein one side of the mouthpiece interface is connected to the lower housing, and the nebulization outlet is on the other side of the mouthpiece interface.

10. The nebulizing-while-inhaling nebulization and inhalation device according to claim 9, wherein the connection between the lower housing and the mouthpiece interface has a right angle or is rounded.

11. The nebulizing-while-inhaling nebulization and inhalation device according to claim 10, wherein when the connection between the lower housing and the mouthpiece interface is rounded, a chamfer radius is 5-40 mm.

12. The nebulizing-while-inhaling nebulization and inhalation device according to claim 1, further comprising an inhalation assembly, wherein the inhalation assembly is a mouthpiece connected to a mouthpiece interface.
